# EUROPEAN PATENT APPLICATION

(11) **EP 1 227 160 A1**
(43) Date of publication of application: **31.07.2002**
(21) Application number: 01101252.3
(22) Date of filing: 19.01.2001
(51) Int. Cl.: C12Q 1/37

(54) **Compounds modulating sister chromatid separation and method for identifying same**

(71) Applicant: BOEHRINGER INGELHEIM INTERNATIONAL GmbH, 55218 Ingelheim am Rhein (DE)
(72) Inventor: Peters, Jan-Michael, Dr., 2100 Korneuburg (AT); Waizenegger, Irene, 1030 Wien (AT)
(74) Representative: Laudien, Dieter, Dr.

(57) **Abstract**

Screening methods for identifying separase inhibitors based on active forms of separase and compounds identified in such methods

## Description

The invention relates to compounds influencing mitosis and meiosis in eukaryotic cells and methods for identifying such compounds. In particular, the invention largely relates to the treatment and prevention of human conditions by modulating sister chromatid segregation.

A key prerequisite for the successful division of one cell into to two is the duplication and subsequent segregation of the cellular genome into the two forming daughter cells. Duplication of the genome by DNA replication occurs during synthesis (S) phase of the cell cycle, whereas segregation of the duplicated DNA takes place much later during anaphase of mitosis. During S phase, replicated DNA molecules remain physically attached to each other, a phenomenon called cohesion, until they are separated in anaphase. At the beginning of mitosis, the cellular DNA is condensed into chromosomes, in which each of the two replicated DNA molecules are microscopically visible as sister chromatids. To allow the segregation of sister chromatids to the forming daughter cells in anaphase, the cohesion that is holding sisters together has to be dissolved. This process is mediated by a protease, called separin or separase, that is cleaving a complex of chromosomal cohesion proteins (the cohesin complex) that is required to hold sister chromatids together. This cleavage reaction liberates sisters from each other so that they can be pulled towards opposite poles of the diving cells by the spindle apparatus (reviewed by Nasmyth et al., 2000).

Inhibition of cohesin cleavage by separase in experimental systems such as budding yeast or human cultured cells inhibits sister chromatid separation and thus prevents the formation of viable daughter cells (Uhlmann et al., 1999; Uhlmann et al., 2000; Waizenegger et al., 2000).

WO 00/48627 suggests a method for identifying compounds which exert their effect by directly modulating, in particular by inhibiting separase's proteolytic activity, i.e. by being protease inhibitors specific for separase.

The screening method for identifying compounds that have the ability of modulating sister chromatid separation in plant or animal cells as described in WO00/48627 comprises incubating separase, in the presence of the substrate(s) for its proteolytic activity and optionally its co-factor(s), with test compounds and determining the modulating effect of the test compounds on the proteolytic activity of the separase.

It was an object of the invention to gain further insight into the mechanism of sister chromatid separation, in particular to elucidate the mechanism by which separase exerts its proteolytic activity, which has been shown to be involved in this process. The mechanism of separase activation provides the basis for developing improved enzymatic assays to identify modulators, in particular inhibitors of separase, in order to provide drugs that exert their effect by modulating, in particular inhibiting, sister chromatid segregation.

The identification of small molecule inhibitors of separase requires enzyme assays in which the protease activity of separase can be directly measured. Preferably, such assays are adaptable to high throughput formats so that large libraries of chemical compounds can be tested for their ability to inhibit separase. The previously reported experiments demonstrating that separase is associated with a protease activity were performed with separase isolated in small scale by immunoprecipitation or affinity chromatography from either yeast or human cells Uhlmann et al., 2000; Waizenegger et al., 2000). Along these lines, WO00/48627 suggests to use full-length separase, preferably in recombinant form, for performing protease assays.

The protease activity of separase is tightly regulated during the cell cycle, ensuring that the ability of separase to cleave cohesin and thereby to dissolve sister chromatid cohesion is not activated before the transition from metaphase to anaphase. Work in budding yeast and human cells has shown that prior to anaphase separase is inhibited by a protein called securin (Ciosk et al., 1998; Uhlmann et al., 1999; Waizenegger et al., 2000). Securin binds to separase until it is ubiquitinated by the anaphase-promoting complex and subsequently degraded by the 26S proteasome shortly before the onset of anaphase. The destruction of securin is thought to activate separase. In human cells, separase itself is cleaved at the same time as securin is destroyed, resulting in two C-terminal cleavage products called p55 and p60 (Waizenegger et al., 2000). These results suggest that the activity of separase is controlled by both securin destruction and separase cleavage. It is not known however and cannot be concluded from these results, which form of the separase, i.e. the full-length form or the cleavage products of separase, represent the active form of the protease or whether and in which way securin has a potential to contribute to the activation of separase.

To establish a separase protease assay suitable for high throughput format it is essential to know which form of separase represents the active enzyme and how the active form of separase can be obtained.

The present invention provides the first evidence that the cleaved forms of separase represent the active protease.

The present invention further provides evidence that securin inhibits separase by directly binding to it and by preventing the access of substrates to the active site of separase. Finally, the present invention provides evidence that it is the autocatalytic reaction, i.e. the reaction in which separase cleaves itself, which is responsible for the cleavage of separase into its active form.

It was previously reported that active human separase can be obtained by immunoprecipitating separase from human cell extracts (Waizenegger et al., 2000). Briefly, antibodies specific for the C-terminus of separase coupled to beads are incubated in lysates obtained from human cultured cells (for example of the line HeLa) which have previously been arrested in mitosis by treatment with microtubule poisons such as nocodazole. After removal of the cell lysate the antibody beads with bound separase are incubated in mitotic Xenopus laevis egg extracts which are able to ubiquitinate and degrade the securin protein which is bound to separase when it is immunoprecipitated from mitotic human cell lysates. During the incubation in mitotic Xenopus extracts the majority of securin is degraded but in addition a major portion of human separase is also cleaved. After washing away the Xenopus egg extracts the antibody beads with bound separase are incubated with either purified cohesin complexes or with recombinant SCC1, which is the subunit of the cohesin complex that is cleaved by separase. The activity of separase is then monitored by analyzing the cleavage of SCC1, which can be analyzed by SDS gel electrophoresis and subsequent immunoblotting with antibodies to SCC1. If recombinant radioactively labeled SCC1 is used the gels can also be analyzed by autoradiography or Phosphorimaging.

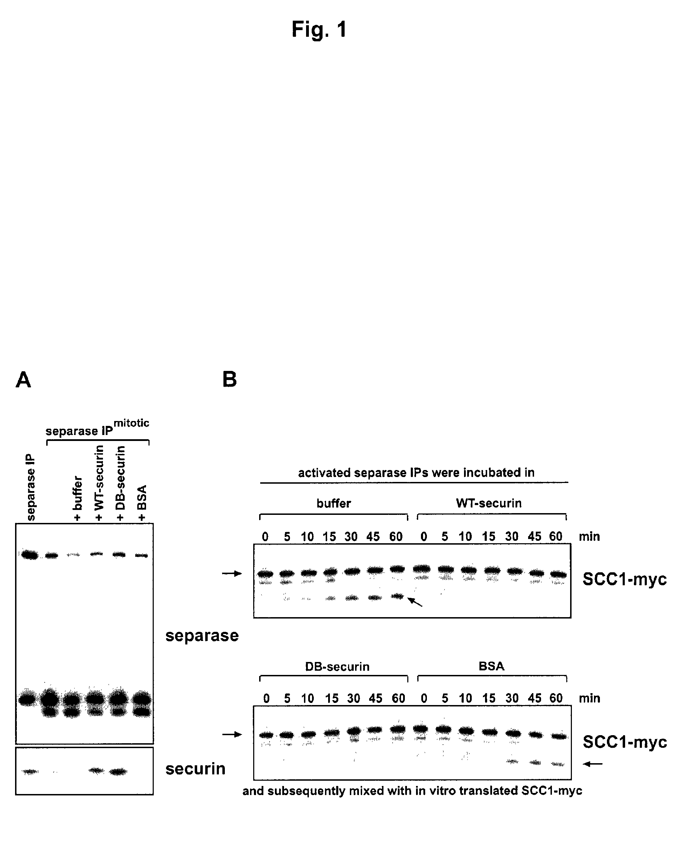

To test if securin inhibits separase by directly binding to it and if it is still able to bind and inhibit the cleaved form of separase human securin was expressed in E. coli and purified. When the recombinant securin was added to separase that had been isolated by immunoprecipitation and had been activated in mitotic Xenopus extracts as above it was observed that securin was able to bind to separase (Figure 1A). Importantly, the amount of securin that bound to separase was at least as high as the amount of securin that had been bound to separase originally before securin was degraded by incubating the separase immunoprecipitates in mitotic Xenopus extracts, although the majority of separase had been cleaved during the incubation in the Xenopus extract. This result shows that securin can bind to the cleaved form of separase as well as to the full-length separase.

When the cleaved form of separase to which recombinant securin had been bound was incubated with purified cohesin complexes no cleavage of the cohesin subunit SCC1 was detected (Figure 1B), demonstrating that recombinant securin is able to inhibit the protease activity of separase by directly binding to it. Because the majority of separase used in this experiment was present in its cleaved form these observations suggest that it is the cleaved form of separase that is the active protease which cleaves SCC1, unless it is inhibited by the rebinding of securin. Identical results were obtained with two different forms of securin, the wildtype protein (WT-securin) and a form that can not be destroyed by ubiquitin-dependent proteolysis (DB-securin; Figure 1A and B).

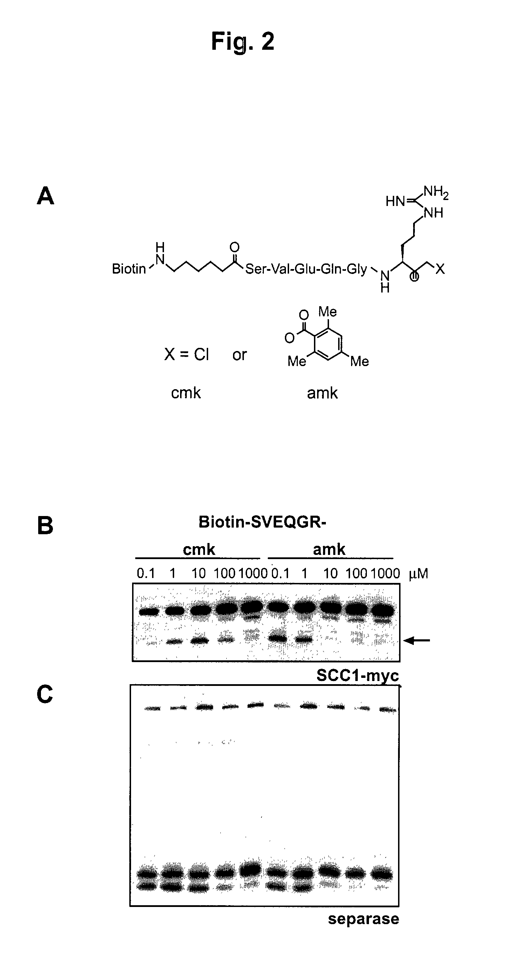

To further study the mechanism of separase activation it was tested if two different peptide inhibitors developed to inhibit separase from budding yeast (Uhlmann et al., 2000) are able to inhibit the protease activity of human separase. These inhibitors are synthetic peptides containing the cleavage site of budding yeast SCC1, "SVEQGR", where the last arginine residue represents the P1 site after which separase cleaves. The C-terminus of this peptide is either modified to a chloromethyl ketone (cmk) or to an acyloxymethyl ketone (amk). Both peptide derivatives are coupled to biotin moieties at their N-termini. The two inhibitors are therefore called Bio-SVEQGR-cmk and Bio-SVEQGR-amk. When these inhibitors were added to human separase that had been isolated by immunoprecipitation and had been activated in mitotic Xenopus extracts as above, it was observed that both inhibitors are able to block the ability of separase to cleave SCC1 (Figure 2A). The concentration of these peptide derivatives required to inhibit human separase was similar to the concentration needed to inhibit separase from budding yeast (compare Fig. 2, upper panel and WO 00/48627, Uhlmann et al., 2000). It was further observed in this experiment that the formation of the p55 cleavage product of human separase was largely inhibited by both peptides at the same concentration at which the ability of separase to cleave SCC1 was inhibited (Figure 2B). The peptide derivatives therefore appear to inhibit separase cleavage which appears to continue during the SCC1 cleavage reaction in the absence of the peptide inhibitors. These observations suggest that separase activity itself is required for separase cleavage, i.e. that separase cleavage occurs autocatalytically.

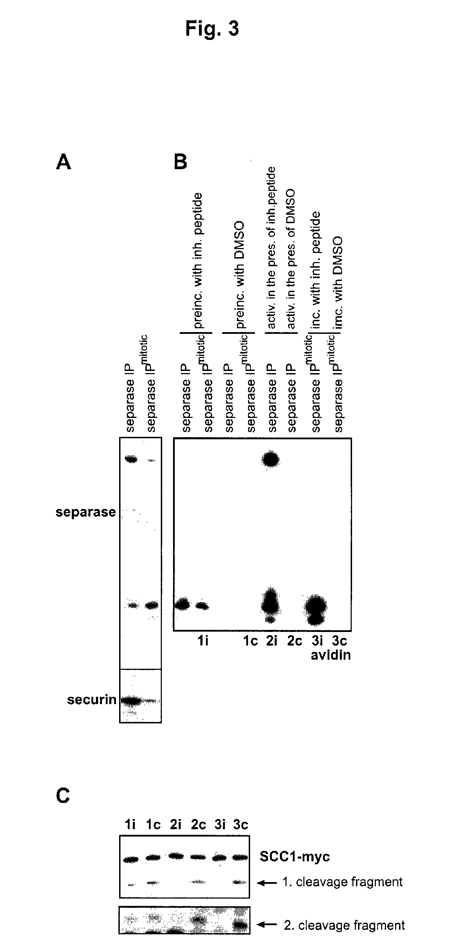

To further test which form of separase represents the active protease, the ability of Bio-SVEQGR-amk, the more effective one of the two peptide inhibitors, to bind to different forms of separase, was tested. Previous work has shown that the peptide derivatives inhibit separase by covalently binding to an active site cysteine residue within separase (Uhlmann et al., 2000). These binding reactions can be directly visualized by separating the separase-inhibitor conjugate by SDS gel electrophoresis and by subsequently labeling the biotin moiety on the peptide derivative by streptavidin (Uhlmann et al., 2000). By using this method it was found that Bio-SVEQGR-amk bound exclusively to the cleaved forms of human separase when the peptide derivative was added to separase after its activation in mitotic Xenopus extracts (Figure 3B, lane 3i). This treatment inhibited the ability of separase to cleave SCC1 (Figure 3C, lane 3i). This observations shows that the active site of separase is accessible to Bio-SVEQGR-amk in the cleaved, but not in the residual amount of full-length separase, confirming the conclusion from the securin addback experiments that the cleaved forms of separase represent active forms of the protease.

When Bio-SVEQGR-amk was added to human separase immunoprecipitates before separase had been activated in mitotic Xenopus extracts, only the small amount of p60 that is already present in these immunoprecipitates was labeled by the peptides, whereas full-length separase was not (Figure 3B, lane 1i), further confirming that the active site of separase is only accessible in the cleaved forms. When the peptide inhibitor was washed away before the separase was subsequently incubated in mitotic Xenopus extracts separase could be activated normally to cleave SCC1 (Figure 3C, lane 1i). This result suggests that the presence of securin prevents the binding of Bio-SVEQGR-amk to the active site of separase, implying that securin inhibits separase by directly or indirectly blocking the access of substrates to the active site of separase.

When the activation of human separase immunoprecipitates in mitotic Xenopus extracts was carried out in the presence of Bio-SVEQGR-amk both the cleaved forms of separase and full-length separase were covalently labeled with the peptide (Figure 3B, lane 2i) and separase was unable to cleave SCC1 (Figure 3C, lane 2i). This observation suggests that the full-length form of separase is also transiently active, presumably once its bound inhibitor securin has been destroyed, but that this form is normally labile because it is further processed into the cleaved forms p55 and p60 by autocleavage.

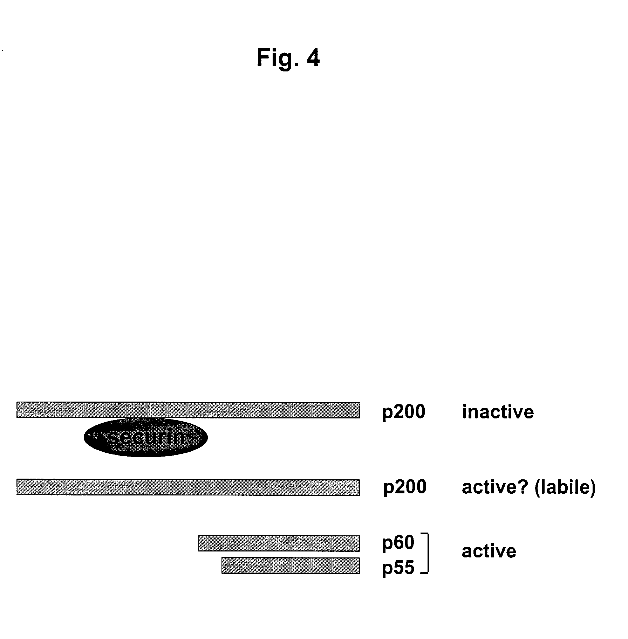

Together, these results suggest the model for the activation of human separase that is shown in Figure 4. According to this model, full-length separase is kept inactive by binding to securin. Following destruction of securin by ubiquitin-dependent proteolysis an active full-length form of separase is generated. This form is very short-lived, however, because it cleaves itself into the mature p55 and p60 forms of separase which are active and long-lived enough to initiate sister chromatid separation by cleaving the SCC1 subunit of cohesin.

The results show that protease assays for identifying separase inhibitors can be based on an active form of human separase, which is present in the cleaved forms of separase. These forms have the advantage to be more stable than the complete separase molecule and are thus expected to be better suitable for being employed in a high throughput format assay.

In order to obtain one or more active, stable forms of separase, the following steps can be taken:

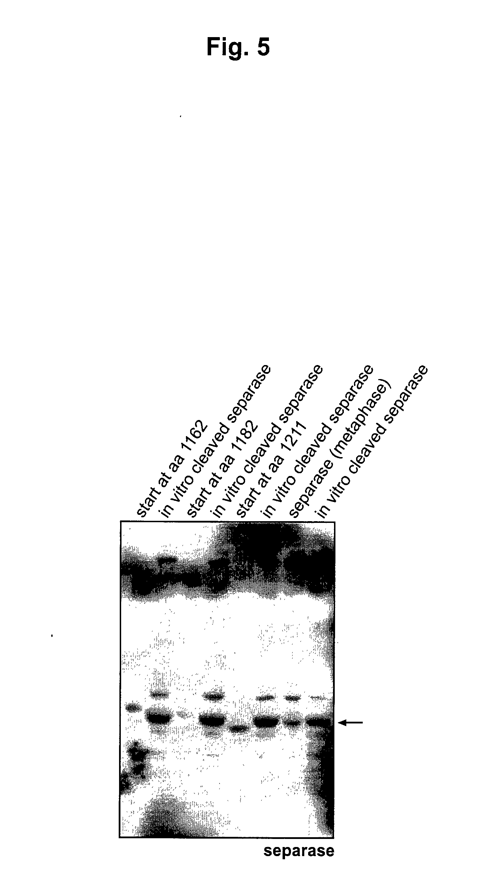

First, it is to be determined if the C-terminal cleavage fragment(s) of human separase that contain(s) the active site alone is/are sufficient for proteolytic activity. To test this possibility the cleavage sites in human separase are mapped, e.g. by a method that has been previously used to map cleavage sites in SCC1 (WO00/48627). Briefly, truncated versions of the human separase cDNA are generated, preferably by polymerase chain reactions (PCR), and the resulting cDNAs are used directly for coupled in vitro transcription-translation reactions, e.g. using rabbit reticulocyte lysates. The transcription-translation reactions are carried out in the presence of labeled amino acids, e.g. ³⁵S-labeled methionine and cysteine, resulting in radiolabeled translation products. These are then separated, e.g. by SDS gel electrophoresis side by side with the in vivo cleavage products of human separase which can be detected by immunoblotting, e.g. like in the experiment shown in Figure 5. The comparison of the electrophoretic mobility of a series of deletion mutants with the mobility of the in vivo cleavage products narrows down the regions of cleavage to about 10 to 20 amino acid residues. Because separase cleavage sites in all known organisms cleave after the sequence EXXR (where X represents any amino acid residue; WO 00/48627 ; Uhlmann et al., 1999; Buonomo et al., 2000) cleavage sites in these regions can be identified by mutating individual arginine residues to alanine or other amino acid residues and by then testing the cleavability of these mutants after expression as recombinant proteins (for example by in vitro transcription-translation).

Once the separase cleavage sites are defined, recombinant forms of the p55 and p60 cleavage products (Waizenegger et al., 2000), or potential other active cleavage products of separase, are produced in suitable expression systems, purified and tested for their ability to cleave SCC1, or a fragment thereof that contains the separase cleavage site, in vitro. Standard expression systems such as E. coli, budding yeast, Baculovirus infected Sf9 and Hi5 insect cells and transfected mammalian, e.g. human cells can be used. For purification, standard biochemical protocols can be used, e.g. those described in WO00/48627 for obtaining separase.

If p55 or p60 (or another, natural or synthetic, C-terminal fragment of human separase) alone or in combination with another C-terminal fragment is sufficient for separase activity, the respective fragment (or a combination of fragments) can be employed in the protease assay (as described in WO00/48627) as a substitute for the full-length separase molecule.

In the case the C-terminal fragment(s) are not sufficient for separase activity, it is next tested if the N-terminal cleavage products, i.e. the N-terminally remaining part(s) of separase, are non-covalently associated with the C-terminal fragments following cleavage of the full-length precursor. To this end, forms of full-length separase that contain epitope tags at their N-termini are generated, in parallel with antibodies specific for the N-terminus of full-length separase. Using standard co-immunoprecipitation experiments, it can be determined if the N- and the C-terminal fragments remain associated with each other.

If this is the case and if the C-terminal fragment(s) alone do not exhibit protease activity, both the N- and the corresponding C-terminal fragments are expressed as recombinant proteins as described above. The two fragments can either be expressed individually, purified and then mixed together, or they can be co-expressed in expression systems as listed above, and the obtained complexes containing both N- and C-terminal fragments are purified. All of these forms, e.g. all combinations of C-terminal and corresponding N-terminal fragments, will then be tested for their ability to cleave SCC1 in vitro. If any of the complexes described above yields human separase activity, the respective complex of separase fragments can be employed in the proteolytic assay in the same manner as a C-terminal fragment by itself, as described above.

If the above-described approach using complexes comprising various separase fragments does not exhibit human separase activity, full-length human separase is expressed in expression systems as listed above, the recombinant protein is isolated and activated, e.g. by incubation in mitotic Xenopus egg extracts, to induce its activation by cleavage.

In parallel, complexes of separase and securin can be generated to investigate whether the binding of securin to separase may not only inhibit separase but may also be required for its subsequent activation. To test this possibility, either recombinant securin is added to recombinant separase after their individual expression and purification, or securin and separase are co-expressed in expression systems as above. All forms of separase, i. e. full-length separase or fragments or fragment combinations, with and without transiently bound securin, are tested for their ability to cleave SCC1 in vitro after incubation of the different forms of separase in mitotic Xenopus extracts.

In the case that these experiments result in the finding that securin is required for human separase activity, the assay is performed by employing the respective form of separase (fragment(s)) in combination with securin. The separase (fragment) is activated in the presence of securin in cell extracts, e.g. Xenopus laevis cell extracts. Preferably, as for the other assay components, securin, or a fragment thereof that proves to be sufficient for activation of separase (if not stated otherwise, "securin" also stands for an active fragment thereof) is employed in recombinant form, based on the cDNA sequence (Lee et al., 1999; Zhang et al, 1999). In order to obtain a protein complex for the assay, the separase (fragment) and securin can either be expressed and purified separately and then combined or they can be co-expressed and co-purified; as described above.

Once the active form(s) of human separase have been obtained by one or more of the methods described above, synthetic peptide substrates for separase are designed and synthesized that allow the simple detection of protease activity in high throughput format, e.g. by fluorogenic methods. The proteolytic assays suitable for this purpose have been described in WO00/48627. By way of example, substrate peptides containing the separase recognition sequence (see WO00/48627) that carry a C-terminal fluorophore such as a 7-amino-4-methyl-coumarin group (AMC) are synthesized by standard methods. The cleavage of AMC (or other fluorophore groups used) results in a rise in fluorescence which can be measured fluorometrically. For high throughput screening methods, compounds, e.g. from chemical or natural product libraries, are next tested for their ability to inhibit the cleavage of fluorogenic peptide substrate by the active form(s) of human separase, which is preferably employed in the screen in recombinant form.

Similarly, the approaches as described above can be used to determine the active forms of separase from other eukaryotic organisms, to generate these forms as recombinant active proteins and to establish screening method for identifying inhibitors of these enzymes.

The present invention relates to method for identifying a compound that has the ability of modulating sister chromatid separation by inhibiting the proteolytic activity of separase, characterized in that an active separase in the form of
a) one or more separase fragment(s), optionally upon activation in the presence of securin, or
b) full-length separase upon activation in the presence of securin,
is incubated in the presence of a separase substrate, with a test compound and that the modulating effect of the test compounds on the proteolytic activity of the separase is determined.

Any variation of the proteolytic screening assay method of the invention, e.g. carried out with one or more separase fragments, in the presence or absence of securin, can be carried out according to standard methods, in particular as described in WO 00/48627.

Starting from the recombinant active separase, alternately to identifying small molecules in a screening method, synthetic peptide derivatives, exemplified by Bio-SVEQGR-amk, can be used as the structural basis to develop peptidomimetic molecules that inhibit separase. For inhibitors of human separase, instead of the cleavage site of budding yeast SCC1 the cleavage sequence of human SCC1 or human separase can be used. The assays described above using recombinant active separase and peptide substrates, e.g. fluorogenic peptides, can be used to optimize such compounds.

Inhibitors of human separase activity identified in the screening methods of the invention or based on rational inhibitor design can be used as cytotoxic therapeutics for the treatment of diseases that are caused by uncontrolled cell proliferation, such as cancers, leukaemias, or cardiac restenosis. Species specific inhibitors of separase from eukaryotic pathogenic microorganisms can be used to treat infectious diseases caused by such microorganisms, for example infections caused by pathogenic fungi or diseases caused by parasites such as Leishmania species. Furthermore, the inhibitors identified in any of the screening methods of the invention or based on rational design can be used in applications described in WO 00/48627.

In a further aspect, the invention relates to a pharmaceutical composition which contains, as the active ingredient, one or more compounds which interfere with or modulate sister chromatid separation by inhibiting the proteolytic activity of separin and that have been identified in any of the screening methods of the present invention or are based on rational design.

### Brief description of the Figures:

- Fig. 1:: Securin acts as an inhibitor for separase
- Fig. 2:: Yeast inhibiting peptides inhibit proteolytic activity of human separase in similar concentration as they inhibit yeast separase and influence the processing of human separase
- Fig. 3:: Addition of yeast inhibiting peptides at different stages during the activation of separase suggests that the cleaved forms are the active forms of separase
- Fig. 4:: Working model for the activation of human separase
- Fig. 5:: Mapping of the cleavage sites in human separase

### Material and methods:

### SCC1 in vitro cleavage assay

Inactive human separase (hseparase) immunoprecipitates were activated in mitotic Xenopus egg extracts (for details see Waizenegger et al., 2000). 30 microliter beads were incubated with 40 µg bacterially expressed wildtype hsecurin, destruction box deleted form of hsecurin (Gmachl et al., 2000) or BSA diluted in XB + 1 mM DTT for 40 min at RT. Subsequently the beads were washed with XB + 1 mM DTT and with TBS + 0.5 M NaCI + 1 mM DTT + 0.5% TWEEN20, followed by one wash with XB + 1mM DTT. To control the rebinding of securin an aliquot of 5 µl was taken per assay and subsequently analysed by immunoblotting with antibodies against separase and securin.

The beads were eventually used for the SCC1 in vitro cleavage assay: 20 µl beads were mixed with 30 µl of the following SCC1 in vitro translation (IVZ) mix (15 µl hSCC1myc IVT + 1 µl PLK-GST + 0.3 µl 1 M MgCl₂, 0.3 µl 100 mM ATP, 0.12 µl 250 mM EGTA, 13.78 µl XB + 1mM DTT) and incubated at 22° C and 1200 rpm. 5 µl were taken per time point, the reaction was stopped by addition of SDS loading buffer. Samples were analyzed by immunoblotting with mouse monoclonal antibodies against myc (9E10).

### Modifications:

A. Mitotically activated separase immunoprecipitates were preincubated with yeast inhibiting peptides (Uhlmann et al., 2000) before they were used in the SCC1-cleavage assay. Biotin-SVEQGR-amk or Biotin-SVEQGR-cmk were used at different concentration (0.1, 1, 10, 100, 1000 µM in XB+ 0.5 mM DTT). Separase immunoprecipitates were incubated for 10 minutes, 22° C and 1200 rpm. The SCC1 in vitro cleavage assay was performed after washing once with XB + 0.5 mM DTT (see above).
B. Separase immunoprecipitates were incubated with 100 µM Biotin-SVEQGR-amk or with DMSO prior mitotic activation. Incubation for 10 minutes, 22° C and 1000 rpm. After washing twice in XB + 1mM DTT the activation was performed in mitotic Xenopus egg extracts.

Interphase Xenopus egg extracts were driven into mitosis in the presence of 1 mM Biotin-SVEQGR-amk or DMSO. Those mitotic Xenopus egg extracts were used to activate a batch of separase immunoprecipitates.

Biotin labeled peptides were detected via immunoblotting according to Faleiro et al., 1997.

### Mapping of cleavage recognition sites in hseparase

N-terminally truncated hseparase cDNA was generated by polymerase chain reaction. The 5'primers contain a sequence with the T7 polymerase binding site
1. start aa1162. 
5'GAATTCTAATACGACTCACTATAGGATCCATGATCCCTGAGGAAGAACTG
ACTG3' (SEQ ID NO:1)
2. start aa1182 
5'GAATTCTAATACGACTCACTATAGGATCCATGTCTGACGGGGAAGACTC
AGCCTC3' (SEQ ID NO:2)
3. start aa 1211

5'GAATTCTAATACGACTCACTATAGGATCCATGGATTCCAGCAAGAAGAAG
CTGCCC3' (SEQ ID NO:3)

The following 3'primer was used:

5'TTATTACCGCAGAGAGACAGGCAAGCC3' (SEQ ID NO:4)

The PCR products were in vitro transcribed and translated with the TNT system (Promega). The obtained in vitro translation products were loaded side by side with in vivo cleaved hseparase on a SDS-PAGE. For immunoblotting the mouse anti-hseparase antibody (7A6) was used.

### Example 1

### Securin acts as an inhibitor for separase

Fig. 1A: Separase immunoprecipitates (separase IP) obtained from Nocodazole arrested HeLa cells and bound by antibodies against the C-terminus of hseparase coupled to beads were activated in mitotic Xenopus egg extracts (separase IP^{mitotic}). Mitotically activated separase immunoprecipitates were either incubated in buffer, wildtype securin, destruction box deleted securin or in BSA. Aliquots were taken and analysed by immunoblotting with antibodies against separase and securin.

Fig.1B: Activated separase immunprecipitates which were either incubated with buffer, wildtype securin, destruction box deleted securin or BSA were incubated SCC1-myc reaction mix. Aliquots were taken at indicated timepoints and analysed by immunoblotting with antibodies against myc. Cleaved SCC1 is marked by arrows.

### Example 2

Yeast inhibiting peptides inhibit proteolytic activity of human separase in similar concentration as they inhibit yeast separase and influence the processing of human separase

Fig. 2A: The structure of the yeast inhibiting peptides

Fig. 2B: Separase immunoprecipitates obtained from Nocodazole arrested HeLa cells bound by antibodies against the C-terminus of hseparase coupled to beads were activated in mitotic Xenopus egg extracts. Subsequently samples were incubated with indicated concentrations of yeast inhibitin peptides (Biotin-SVEQGR-cmk or Biotin-SVEQGR-amk). After a short wash samples were mixed with SCC1-myc reaction mix for 1 hour. Samples were analysed by immunoblotting with antibodies against myc. Cleaved SCC1 is marked by an arrow.

Fig. 2C: The samples (see B) were immunblotted with antibodies against separase.

### Example 3

Addition of yeast inhibiting peptides at different stages during the activation of separase suggests that the cleaved forms are the active forms of separase

Fig. 3A: Separase immunoprecipitates (separase IP) obtained from Nocodazole arrested HeLa cells bound by antibodies against the C-terminus of hseparase coupled to beads were activated in mitotic Xenopus egg extracts (separase IP^{mitotic}). Aliquots were analysed by immunoblotting with antibodies against separase and securin.

Fig. 3B: Separase IPs (see A) were either preincubated with Biotin-SVEQGR-amk (preinc. with inh. peptide) or with DMSO (preinc. with DMSO), subsequently washed and aliquots were taken. Thereafter they were incubated in mitotic Xenopus egg extracts and again washed. Samples were taken for analysis (1i, 1c). Separase IPs were activated either in mitotic Xenopus egg extracts which were driven into mitosis in the presence of Biotin-SVEQGR-amk or DMSO, subsequently washed and aliquots were taken for analysis (2i, 2c). Already mitotically activated separase IPs (see A) were incubated with Biotin-SVEQGR-amk or DMSO, thereafter washed and aliquots were taken for analysis (3i, 3c). All samples were analysed by immunoblotting with avidin.

Fig. 3C: Samples 1i, 1c, 2i, 2c, 3i and 3c (see B) were mixed with SCC1-myc reaction mix for 1 hour and analysed by immunoblotting with antibodies against myc. Arrows indicate the first and second SCC1 cleavage product.

### Example 4

### Working model for the activation of separase (Fig. 4)

Full-length separase (p200) is associated with securin and represent the inactive state of the protease. Upon securin proteolysis of securin a labile full-length form of separase exists. The cleaved forms of separase (p55 and p60) represent the stable, active enzyme. If they act together and are associated with N-terminally cleaved forms is yet to be further analysed.

### Example 5

### Mapping of the hseparase recognition sites

N-terminally truncated hseparase cDNA was in vitro transcribed and translated. The recombinant products start with an exogenous methionine at the indicated amino acids. The recombinant products were loaded on an SDS-PAGE alternating with total extracts from HeLa cells arrested with Nocodazole and released into the cell cycle for 2,5 hours (in vitro cleaved separase). As a control a total extract from HeLa cells arrested with Nocodazole was loaded (separase (metaphase)). Immunoblotting was performed with antibodies against separase. Cleaved separase is indicated by an arrow (Fig. 5).

### References

Buonomo SB, Clyne RK, Fuchs J, Loidl J, Uhlmann F, Nasmyth K; Cell 2000 Oct 27;103(3):387-98

Ciosk R, Zachariae W, Michaelis C, Shevchenko A, Mann M, Nasmyth K; Cell 1998 Jun 12;93(6):1067-76

Faleiro L, Kobayashi R, Fearnhead H, Lazebnik Y; EMBO J 1997 May 1;16(9):2271-81

Gmachl M, Gieffers C, Podtelejnikov AV, Mann M, Peters JM; Proc Natl Acad Sci U S A 2000 Aug 1;97(16):8973-8

Lee IA, Seong C, Choe IS; Biochem Mol Biol Int 1999 May;47(5):891-7

Nasmyth K, Peters JM, Uhlmann F; Science 2000 May 26;288(5470):1379-85

Uhlmann F, Wernic D, Poupart MA, Koonin EV, Nasmyth K; Cell 2000 Oct 27;103(3):375-86

Uhlmann F, Lottspeich F, Nasmyth K; Nature 1999 Jul 1;400(6739):37-42

Waizenegger IC, Hauf S, Meinke A, Peters; JM Cell 2000 Oct 27;103(3):399-410

Zhang X, Horwitz GA, Heaney AP, Nakashima M, Prezant TR, Bronstein MD, Melmed S; J Clin Endocrinol Metab 1999 Feb;84(2):761-7

## Claims

1. A method for identifying a compound that has the ability of modulating sister chromatid separation by inhibiting the proteolytic activity of separase, **characterized in that** an active separase in the form of
a) one or more separase fragment(s), optionally upon activation in the presence of securin, or
b) the full-length separase upon activation in the presence of securin,
is incubated in the presence of a separase substrate, with a test compound and that the modulating effect of the test compound on the proteolytic activity of the active separase is determined.

2. The method of claim 1, wherein the active separase is human.

3. The method of claim 1 or 2, wherein the active separase (fragment) is activated in a mitotic cell extract in the presence of securin.

4. The method of claim 3, wherein the mitotic cell extract has been obtained from Xenopus laevis eggs.

5. An inhibitor of separase which has been identified in the method of claim 1 for human therapy.


SEQUENCE LISTING

<110> Boehringer Ingelheim International GmbH

<120> Compounds modulating sister chromatid separation and method for
      identifying same

<130> Case14/060

<140>
<141>

<160> 4

<170> PatentIn Ver. 2.1


<210> 1
<211> 54
<212> DNA
<213> Artificial Sequence

<220>
<223> Description of Artificial Sequence: Primer

<400> 1
gaattctaat acgactcact ataggatcca tgatccctga ggaagaactg actg       54


<210> 2
<211> 55
<212> DNA
<213> Artificial Sequence

<220>
<223> Description of Artificial Sequence: Primer

<400> 2
gaattctaat acgactcact ataggatcca tgtctgacgg ggaagactca gcctc      55


<210> 3
<211> 56
<212> DNA
<213> Artificial Sequence

<220>
<223> Description of Artificial Sequence: Primer

<400> 3
gaattctaat acgactcact ataggatcca tggattccag caagaagaag ctgccc     56


<210> 4
<211> 27
<212> DNA
<213> Artificial Sequence

<220>
<223> Description of Artificial Sequence: Primer

<400> 4
ttattaccgc agagagacag gcaagcc                                     27